(19) Europäisches Patentamt
European Patent Office
Office européen des brevets

(11) **EP 4 579 209 A1**

(12) **EUROPEAN PATENT APPLICATION**
published in accordance with Art. 153(4) EPC

(43) Date of publication:
**02.07.2025 Bulletin 2025/27**

(21) Application number: **23871074.3**

(22) Date of filing: **28.09.2023**

(51) International Patent Classification (IPC):
*G01N 1/14* (2006.01)

(52) Cooperative Patent Classification (CPC):
**G01N 1/14**

(86) International application number:
**PCT/CN2023/122840**

(87) International publication number:
**WO 2024/067850 (04.04.2024 Gazette 2024/14)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 30.09.2022  CN 202211219926
30.09.2022  CN 202211219890
30.09.2022  CN 202211219889

(71) Applicant: **Beijing Sightnovo Medical Technology
Co., Ltd
Haidian District Beijing 100192 (CN)**

(72) Inventors:
• XIA, Chaoran
  **Beijing 100192 (CN)**
• ZHONG, Weixing
  **Beijing 100192 (CN)**
• ZHANG, Miao
  **Beijing 100192 (CN)**

(74) Representative: **Gille Hrabal
Partnerschaftsgesellschaft mbB
Patentanwälte
Brucknerstraße 20
40593 Düsseldorf (DE)**

(54) **TEAR SAMPLE ELUENT, DILUENT AND PREPARATION METHOD THEREFOR, AND TEAR COLLECTION AND TREATING APPARATUS**

(57) Disclosed is a method for preparing a tear diluent, and provided are a tear sample eluent and a tear sample collection and treating apparatus. A small-volume tear sample can be stably collected using an absorbent material, and a substance to be tested of the sample can be stably released after elution using the sample eluent provided in the present disclosure.

FIG. 1

## Description

The present disclosure claims priority to:

the prior Patent Application No. 2022112199260 entitled "TEAR SAMPLE COLLECTION AND TREATING DEVICE" filed with the China National Intellectual Property Administration on Sep. 30, 2022;
the prior Patent Application No. 2022112198906 entitled "TEAR SAMPLE ELUTION SOLUTION AND USE THEREOF" filed with the China National Intellectual Property Administration on Sep. 30, 2022; and
the prior Patent Application No. 2022112198893 entitled "METHOD FOR PREPARING TEAR DILUTION SOLUTION" filed with the China National Intellectual Property Administration on Sep. 30, 2022,
the contents of which are incorporated herein by reference in their entireties.

## TECHNICAL FIELD

The present disclosure relates to the field of biological sample collection, and particularly, to a tear sample elution solution, a tear dilution solution and a method for preparing same, and a tear collection and treating device.

## BACKGROUND

Tears are weakly alkaline, transparent liquids primarily containing water, along with inorganic salts and bioactive molecules such as proteins, lipids, lysozymes, the complement system, and other substances, making it a highly complex system. Tears are evenly distributed within the conjunctival sac, forming a liquid film known as the tear film. The portion of the tear film located in front of the cornea is commonly referred to as the precorneal tear film (PCTF). Despite its thickness of only 6 nm to 10 nm, the precorneal tear film is able to maintain the moisture of the eyeball and improve the refraction of eye by filling the micro-undulations on the cornea.

The secretion status of the human precorneal tear film is influenced by the metabolic state of the ocular surface, and the presence of ocular surface diseases and the homeostasis of the human body can be detected by analyzing the composition of the precorneal tear film. Therefore, biochemical analysis results of PCTF components hold the potential for detecting the presence or progression of diseases and for the application to clinical treatment. Moreover, since tears exist on the body surface (the ocular surface), their collection is non-invasive, offering a more comfortable, hygienic, and convenient option for subjects compared with collecting blood, urine, and stool samples.

However, the volume of tear samples that can be collected is significantly smaller than those of conventional clinical samples such as blood, urine, and stool samples. This results in poor detection rates, accuracy, reproducibility, and reliability of detection results, or necessitates substantial efforts to ensure reliable results.

In addition, existing methods for collecting tear samples primarily include the stimulated tear collection method and the capillary tube tear collection method. The former requires stimulating tear secretion, typically by applying irritants such as mentholated balm to the lower eyelid or acupuncturing at the Jingming point. This method easily causes discomfort to the subject and leads to varied sample volumes, resulting in highly fluctuating detection results. The latter requires the use of a capillary tube to draw tears from the ocular surface after reflex tears appear in the eye. This method imposes extremely high requirements on the operator to avoid injuring the subject's eye. Additionally, due to the limited capacity of the capillary tube, the collected sample volume is insufficient, which is unfavorable for subsequent detection. Moreover, the use of irritants in the stimulated tear collection method poses a risk of sample contamination, thereby distorting the real condition of the tears.

Meanwhile, current immunological detection techniques, including fluorescent immunochromatography, colloidal gold immunochromatography, magnetic particle immunochemiluminescence, latex microsphere immunochromatography, and enzyme-linked immunosorbent assay (ELISA), require large-volume samples and the coordination of a number of independent instruments and apparatuses to give reliable results. These methods are cumbersome in procedures, highly demanding for the skills of operators, and time-consuming in process, which limits their applications to a small-volume sample. If a small-volume sample is used in these methods, the reliability of results is compromised, making it difficult to provide a clinically meaningful basis for judgment.

Therefore, there is an urgent need in the field for reliable means to collect and process a small-volume tear sample.

## SUMMARY

In view of the problems in the prior art, the inventors have found that a small-volume tear sample can be stably collected using an absorbent material, and the substance of interest in the sample can be stably released after elution using a sample elution solution of a specific composition. This allows for acquiring a qualitative, semi-quantitative, and quantitative analysis that can be further used for the substance of interest. In an additional step, the analysis of various physiological parameters of a subject can also be achieved by means of the analysis results of different substances of interest. Thus, the present disclosure is completed.

Therefore, in a first aspect, provided herein is a tear sample elution solution comprising water as a solvent, a buffering agent, a surfactant, a stabilizer, a preservative, and a tonicity adjusting agent, and having a pH value of 7.0 to 9.0.

In one embodiment, a pH regulator is optionally used to adjust the sample elution solution to a pH value of 7.0 to 9.0. Preferably, the pH regulator comprises an organic or inorganic acid such as hydrochloric acid, sulfuric acid, or phosphoric acid; or an organic or inorganic base such as sodium hydroxide or potassium hydroxide. The content of the pH regulator is not particularly limited, and the concentration and amount of the pH regulator can be selected by those skilled in the art according to the final pH value of the sample elution solution, thereby achieving the final desired pH value of the sample elution solution. In one embodiment, the sample elution solution is weakly alkaline. For example, the sample elution solution has a pH value of about 7.1 to 8.5, preferably 7.5 to 8.2, and more preferably 7.8 to 8.1, e.g., about 7.4, 7.6, or 8.0. Preferably, the final pH of the sample elution solution is adjusted using an HCl solution.

In one embodiment, the buffering agent is selected from an organic amine buffering agent, preferably an organic amine buffering agent with a hydroxyl group. As an example, the buffering agent may be selected from tris(hydroxymethyl) aminomethane, i.e., trometamol (Tris), and/or tris(hydroxymethyl)aminomethane-hydrochloride (Tris-HCl). In the sample elution solution, the content of the buffering agent may range from 10 mM to 100 mM, preferably from 20 mM to 80 mM, and more preferably from 35 mM to 60 mM, particularly, about 50 mM.

In one embodiment, a combination of tris(hydroxymethyl)aminomethane as a buffering agent and HCl as a pH regulator is preferred.

In one embodiment, the surfactant may comprise a first surfactant and a second surfactant, which may be identical or different. Preferably, the surfactant is selected from a nonionic surfactant. In one embodiment, the first surfactant is different from the second surfactant. Preferably, the first surfactant is selected from a polysorbate surfactant, more preferably a surfactant from the TWEEN series, including but not limited to TWEEN 20 and TWEEN 80. Preferably, the second surfactant is selected from a block polyether surfactant, such as a polyethylene glycol surfactant with a terminal hydroxyl group, more preferably a polyethylene glycol phenyl ether surfactant. As an example, the second surfactant may be polyethylene glycol mono-4-octylphenyl ether, which is commercially available under the name Triton™. Alternatively, it may be a polymer surfactant composed of methyl oxirane, 1,2-ethylenediamine, and oxirane, which is commercially available under the name Tetronic 1307 (S9). In the sample elution solution, the content of the surfactant may range from 0.1 wt% to 5 wt%, preferably from 0.5 wt% to 4 wt%, and more preferably from 1 wt% to 3.5 wt%. The weight ratio of the first surfactant to the second surfactant may range from 10:1 to 1:10, preferably from 1:1 to 1:10, and more preferably from 1:1 to 1:8, e.g., about 1:5. In one embodiment, the first surfactant is different from the second surfactant, wherein the first surfactant is selected from a surfactant from the TWEEN series and the second surfactant is selected from a surfactant from the Triton series. A combination of TWEEN 20 and Triton X-100 is preferred, and the concentration ratio of TWEEN 20 to Triton X-100 is preferably about 1:1, 1:2, 1:5, or 1:10. In the most preferred embodiment, the sample elution solution comprises about 0.5 wt% of TWEEN 20 and about 2.5 wt% of Triton X-100. In one embodiment, the stabilizer is selected from albumin, casein, gelatin. Preferably, the stabilizer is selected from casein. In the sample elution solution, the content of the stabilizer may range from 0.1 wt% to 5 wt%, preferably from 0.5 wt% to 4 wt%, and more preferably from 0.8 wt% to 2 wt%., e.g., about 1 wt%.

In one embodiment, the sample elution solution does not comprise particles having a particle size of larger than 0.50 μm, preferably larger than 0.45 μm, and more preferably larger than 0.40 μm.

In one embodiment, the preservative is selected from Proclin-300, sodium azide, thiomersal. In the sample elution solution, the content of the preservative may range from 0.01 wt% to 0.5 wt%, preferably from 0.05 wt% to 0.4 wt%, and more preferably from 0.1 wt% to 0.3 wt%, e.g., 0.1 wt%, 0.2 wt%, or 0.3 wt%.

In one embodiment, the tonicity adjusting agent is an alkali metal salt, such as an inorganic or organic salt; preferably a sodium or potassium salt; and more preferably NaCl.

In one embodiment, the sample elution solution may optionally comprise an organic solvent. Preferably, the sample elution solution does not comprise an organic solvent. In a preferred embodiment, the sample elution solution comprises only water as a solvent.

In one embodiment, the sample elution solution may comprise about 10 mM to 100 mM of a buffering agent, about 0.1 wt% to 5 wt% of a surfactant, about 0.1 wt% to 5 wt% of a stabilizer, and optionally about 0.01 wt% to 0.5 wt% of a preservative and about 0.1 wt% to 5 wt% of a tonicity adjusting agent; preferably, the elution solution has a pH value of about 7.0 to 9.0.

In one embodiment, the sample elution solution may comprise about 35 mM to 60 mM of a buffering agent, about 1 wt% to 3.5 wt% of a surfactant, about 0.8 wt% to 2 wt% of a stabilizer, and optionally about 0.1 wt% to 0.3 wt% of a preservative and about 0.5 wt% to 1.2 wt% of a tonicity adjusting agent, wherein the surfactant comprises a first surfactant and a second surfactant, and the weight ratio of the first surfactant to the second surfactant may range from 10:1 to 1:10, preferably from 1:1 to 1:10, and more preferably from 1:1 to 1:8; preferably, the elution solution has a pH value of about 7.4 to 8.1.

In one embodiment, the sample elution solution may comprise about 50 mM of a buffering agent, about 0.5 wt% of a first surfactant, about 2.5 wt% of a second surfactant, about 1 wt% of a stabilizer, and optionally about 0.2 wt% of a preservative and about 0.9 wt% of NaCl; preferably, the elution solution has a pH value of about 7.4.

As an example, the sample elution solution may comprise about 50 mM of a Tris buffering agent, about 0.5% of TWEEN 20, about 2.5 wt% of Triton X-100, about 1 wt% of casein, and optionally about 0.2 wt% of Proclin-300 and about 0.9 wt% of NaCl; preferably, the elution solution has a pH value of about 7.4.

As an example, the sample elution solution comprises about 50 mM of a Tris buffering agent, about 0.5% of TWEEN 20, about 2.5 wt% of Triton X-100, about 1 wt% of casein, and about 0.2 wt% of Proclin-300 and about 0.9 wt% of NaCl; preferably, the elution solution has a pH value of about 7.4. In one embodiment, the sample elution solution is used to elute tears adsorbed by a first absorbent sheet. In another embodiment, the first absorbent sheet and the sample elution solution compose a tear sample collection and treating device.

In one embodiment, the first absorbent sheet may have one or more staining indicator line layers. The staining indicator line layer may comprise a fluorescent substance such as sodium fluorescein. Preferably, the first absorbent sheet is a filter paper strip. As an example, the first absorbent sheet is, for example, a detection filter paper strip for the Schirmer tear secretion test. In one embodiment, the first absorbent sheet also has one, two, or more readable scales starting from an end. The end is preferably a sampling end. The substrate material of the first absorbent sheet is not particularly limited. In one embodiment, the first absorbent sheet is a hydrophilic inert sheet, and its substrate material may comprise cellulose and a polymer, such as polyurethane.

In one embodiment, the length of the first absorbent sheet is at least about 5 mm, 6 mm, 10 mm, 15 mm, or even 20 mm. In one embodiment, the width of the first absorbent sheet is at least about 5 mm, 6 mm, or 7 mm. In one embodiment, the predetermined length is at least about 5 mm, 6 mm, 7 mm, 8 mm, or 10 mm.

In one embodiment, the fully saturated portion of the predetermined length contains tears of about 0.0001 mL to 0.0050 mL, preferably about 0.001 mL to 0.004 mL, more preferably about 0.0015 mL to 0.0025 mL, even more preferably about 0.0018 mL to 0.0020 mL, and most preferably about 0.0020 mL. In the present disclosure, the tears contained in the fully saturated portion of the predetermined length may also be referred to as the "same tear sample", as the first absorbent sheet is for single use, and the tear sample collected during each collection procedure is used for only one detection run.

In one embodiment, the predetermined volume of the sample elution solution is at least about 0.100 mL, 0.150 mL, 0.200 mL, 0.250 mL, 0.300 mL, or 0.500 mL.

In a second aspect of the present disclosure, provided herein is a method for preparing a tear dilution solution, comprising the following steps:

> a. contacting a first absorbent sheet with the ocular surface to collect tears, so as to give a first absorbent sheet at least partially saturated with tears, wherein the first absorbent sheet at least partially saturated with tears has a fully saturated portion of a predetermined length or is fully saturated with tears; and
> b. contacting the fully saturated portion of the predetermined length of the first absorbent sheet with the sample elution solution according to the first aspect of a predetermined volume for a predetermined time to give a tear dilution solution, or immersing the first absorbent sheet fully saturated with tears in the sample elution solution of the predetermined volume to give a tear dilution solution.

In one embodiment, the predetermined time for contacting the first absorbent sheet with the sample elution solution is 1 s to 5 min, preferably 3 s to 3 min, and more preferably 5 s to 1 min. The contact between the first absorbent sheet and the sample elution solution may be achieved by immersion and/or stirring.

In a third aspect of the present disclosure, also provided is an immunological detection kit comprising an immunological detection card, wherein the detection card comprises a support, a blotting membrane fixedly connected to the support, an absorbent pad located at a first end of the blotting membrane, and a sample receiving pad located at a second end of the blotting membrane. The absorbent pad and the sample receiving pad are spatially separated from each other, and the blotting membrane therebetween is provided with a control line proximal to the side of the absorbent pad and a detection line proximal to the side of the sample receiving pad.

In one embodiment, the support is made of at least one material selected from a polymer, glass and polyvinyl chloride (PVC), polystyrene (PS), and a combination thereof.

In one embodiment, the blotting membrane comprises at least one material selected from cellulose, nitrocellulose, and/or a combination thereof.

In one embodiment, the sample receiving pad is a glass fiber membrane, nylon-based cellulose, or polyester cellulose. In one embodiment, the sample receiving pad comprises any one of the following substances: a fluorescent latex microsphere and a colloidal gold probe. Preferably, the fluorescent latex microsphere is a polystyrene latex microsphere conjugated with a fluorescently labeled probe.

In one embodiment, the immunological detection card further comprises a second absorbent sheet. The second absorbent sheet may be identical to or different from the first absorbent sheet in dimension and structure, but comprises a defined content of a target substance. Preferably, the first absorbent sheet and the second absorbent sheet have the same substrate material and dimension.

In one embodiment, the immunological detection includes fluorescent immunochromatography, colloidal gold immunochromatography, latex microsphere immunochromatography, magnetic particle chemiluminescence and/or enzyme-linked immunosorbent assay. In one embodiment, the immunological detection card is a fluorescent immunochromatographic detection card or a colloidal gold immunochromatographic detection card.

In one embodiment, the target substance may include: IL-8, IL-1a, IL-1b, IL-12, IL-6, IL-8, tumor necrosis factor-$\alpha$ (TNF-$\alpha$) and interferon-$\gamma$ (INF-$\gamma$), lysozyme, lactoferrin, epidermal growth factor (EGF), lipocalin-1, cystatin S100, $\alpha$1-antitrypsin, $\alpha$-enolase, $\alpha$-1-acid glycoprotein 1, S100 A8 (calgranulin A), S100 A9 (calgranulin B), S100 A4 and S100 A11 (calcium-binding proteins), prolactin-induced protein (PIP), proline-rich protein 4 (PRR4), PRR3, NACPP4, S100A6, annexin A1 (ANXA1), annexin A11 (ANXA11), cystatin-S (CST4), phospholipase A2 activating protein (PLAA), lactoglobulin B, lipophilin A, matrix metalloproteinase (MMP)-9, anti-Ro/SSA and anti-La/SSB antibodies, anti-$\alpha$-fodrin antibodies, aquaporin 5 (AQP5), complement C-3, albumin, potassium ion, sodium ion, chloride ion.

In one embodiment, the detection line (12) comprises a murine anti-human matrix metalloproteinase-9 IgG; preferably, the concentration of the murine anti-human matrix metalloproteinase-9 IgG in the detection line (12) is about 5.0 mg/mL, 4.0 mg/mL, 3.0 mg/mL, 2.0 mg/mL, 1.0 mg/mL, 0.5 mg/mL, 0.25 mg/mL, or in a range defined by any two of these values as endpoints and any value included therein. In one embodiment, the control line (b) comprises a goat anti-chicken IgY. In one embodiment, the fluorescent latex microsphere comprises a conjugated murine anti-human matrix metalloproteinase-9 IgG. In one embodiment, the detection line (12) comprises a murine anti-human matrix metalloproteinase-9 IgG, the control line (11) comprises a goat anti-chicken IgY, and the fluorescent latex microsphere comprises a conjugated murine anti-human matrix metalloproteinase-9 IgG. Therefore, the immunological detection card can be used to detect human matrix metalloproteinase.

In a fourth aspect, the present disclosure further provides a method for preparing an immunological detection card, comprising the following steps:

(i) providing a blotting membrane with a control line and a detection line, and optionally drying at 37 °C for 8-20 h;
(ii) providing a sample receiving pad and applying a fluorescent latex microsphere or colloidal gold probe to the sample receiving pad, and optionally drying at 37 °C for 8-20 h;
(iii) placing the sample receiving pad at a second end of the blotting membrane proximal to the detection line and bringing the two into contact; and
(iv) placing an absorbent pad at a first end of the blotting membrane proximal to the control line and bringing the two into contact.

In one embodiment, the fluorescent latex microsphere is provided on the sample receiving pad. Preferably, providing the fluorescent latex microsphere on the sample receiving pad comprises the following steps:

(ii-1) treating a blank fluorescent latex microsphere with an activation solution;
(ii-2) treating the fluorescent latex microsphere obtained in step (ii-1) with a coupling buffer to give a fluorescent latex microsphere suspension;
(ii-3) treating the fluorescent latex microsphere suspension obtained in step (ii-2) with a murine anti-human matrix metalloproteinase-9 IgG;
(ii-4) treating the fluorescent latex microsphere suspension obtained in step (ii-3) with a blocking solution;
(ii-5) treating the fluorescent latex microsphere suspension obtained in step (ii-4) with a second washing solution; and
(ii-6) diluting the fluorescent latex microsphere suspension obtained in step (ii-5) with a microsphere dilution solution and then spraying it onto a blank sample receiving pad.

Preferably, prior to step (ii-1), the method further comprises step (ii-0): washing the fluorescent latex microsphere with a first washing solution to give a blank fluorescent latex microsphere. More preferably, the washing is performed under a ultrasonication, and even more preferably, the washing further comprises a centrifugation after the ultrasonication. Preferably, the first washing solution comprises morpholinoethanesulfonic acid (MES), and is preferably an aqueous solution of MES.

Preferably, treating the blank fluorescent latex microsphere with the activation solution in step (ii-1) comprises treating the blank fluorescent latex microsphere with a first activation solution and a second activation solution. The first activation solution is a solution of sulfo-NHS (N-hydroxysulfosuccinimide sodium salt) in the first washing solution, and preferably, the concentration of sulfo-NHS is 10 mg/mL to 30 mg/mL. The second activation solution is a solution of EDC (1-ethyl-(3-dimethylaminopropyl)carbodiimide hydrochloride) in the first washing solution, and preferably, the concentration of EDC is 10 mg/mL to 30 mg/mL. The blocking solution comprises casein, bovine serum albumin, and a buffering agent.

Preferably, the coupling buffer in step (ii-2) comprises N-(2-hydroxyethyl)piperazine-N-2-ethanesulfonic acid at a concentration of about 6 g/L and a pH value of about 8.0.

Preferably, after the treatments in steps (ii-1), (ii-2), (ii-3), (ii-4), and/or (ii-5), the method further comprises a centrifugation, which is preferably performed for 5 min to 60 min, more preferably performed for 10 min to 40 min, and even more preferably performed for 10 min to 30 min. Preferably, the method further comprises a ultrasonication-assisted washing in steps (ii-0) and/or (ii-4).

Preferably, the spraying in step (ii-6) is performed in an amount of 5-10 μL/cm.

Preferably, in step (ii-3), the mass ratio of the murine anti-human matrix metalloproteinase-9 (MMP-9) IgG to the fluorescent latex microsphere suspension obtained in step (ii-2) is (0.1-0.5):(1-5). The treatment in step (ii-3) is performed at 35 °C to 40 °C, preferably performed at 37 °C for at least 2 h, preferably at least 3 h.

Preferably, the treatment in step (ii-4) is performed at 35 °C to 40 °C, preferably performed at 37 °C for 5 min to 60 min, more preferably performed for 10 min to 40 min, and even more preferably performed for 10 min to 30 min.

As an example, the first washing solution is an about 10.66 g/L aqueous solution of morpholinoethanesulfonic acid having a pH of about 6.1; the first activation solution is an about 20 mg/mL solution of sulfo-NHS in the first washing solution; the second activation solution is an about 20 mg/mL solution of 1-ethyl-(3-dimethylaminopropyl)carbodiimide hydrochloride in the first washing solution; the coupling buffer is an about 5.96 g/L solution of N-(2-hydroxyethyl)piperazine-N-2-ethanesulfonic acid having a pH of about 8.0; the blocking solution is a solution containing 30 g/L of casein, 10 g/L of bovine serum albumin, and 0.05 mol/L of a Trisbase buffer; the second washing solution is a 6 g/L Trisbase buffer, about pH 8.0; the microsphere dilution solution is a buffer containing 30% of sucrose, 0.5% of casein, 1% of glycine, and 50 mM of Trisbase having a pH of about 8.0.

In a fifth aspect, further provided herein is a method for detecting a target substance in tears.

In one embodiment, the method for detecting a target substance in tears comprises the following steps:

a. contacting a first absorbent sheet with the ocular surface to collect tears, so as to give a first absorbent sheet at least partially saturated with tears, wherein the first absorbent sheet at least partially saturated with tears has a fully saturated portion of a predetermined length or is fully saturated with tears;

b. contacting the fully saturated portion of the predetermined length of the first absorbent sheet with a sample elution solution of a predetermined volume for a predetermined time to give a tear dilution solution, or immersing the first absorbent sheet fully saturated with tears in the sample elution solution of the predetermined volume to give a tear dilution solution; and

c. using the obtained tear dilution solution for immunological detection to analyze the target substance in the tears.

In one embodiment, step c of the method for detecting the target substance in tears comprises contacting the obtained tear dilution solution with the immunochromatographic detection card according to the third aspect of the present disclosure.

In one embodiment, the method for detecting a target substance in tears comprises the following steps:

a'. contacting a first absorbent sheet with the ocular surface to collect tears, so as to give a first absorbent sheet at least partially saturated with tears, wherein the first absorbent sheet at least partially saturated with tears has a fully saturated portion; and

b'. contacting the first absorbent sheet with the immunological detection card according to the third aspect of the present disclosure.

**In** one embodiment, the method for detecting a target substance in tears comprises the following steps:

a. contacting at least two first absorbent sheets with the ocular surface of the same subject at different time points to collect tears, so as to give at least two first absorbent sheets at least partially saturated with tears, wherein the first absorbent sheet at least partially saturated with tears has a fully saturated portion of a predetermined length or is fully saturated with tears;

b. contacting the fully saturated portion of the predetermined length of each of the first absorbent sheets with a sample elution solution of a predetermined volume for a predetermined time to give a tear dilution solution, or immersing each of the first absorbent sheets fully saturated with tears in the sample elution solution of the predetermined volume to give a tear dilution solution; and

c. using the obtained tear dilution solution for immunological detection to analyze the target substance in the tears collected from the same subject at different time points.

In one embodiment, the method for detecting a target substance in tears comprises the following steps:

a'. contacting at least two first absorbent sheets with the ocular surface of the same subject at different time points to collect tears, so as to give at least two first absorbent sheets at least partially saturated with tears, wherein the first absorbent sheet at least partially saturated with tears has a fully saturated portion; and

b'. contacting the at least two first absorbent sheets separately with the immunological detection card according to the third aspect of the present disclosure.

In one embodiment, the same subject is in different states at different time points, such as a healthy state or a diseased state. Specifically, the subject may be in a healthy state or a diseased state at all time points, or in a healthy state at one time point and a diseased state at another time point. In one embodiment, provided herein is qualitative, semi-quantitative, or quantitative detection of a target substance in one or more tears collected from the same subject at different time points. In one embodiment, provided herein is a quantitative detection of a target substance in a plurality of tear samples from the same subject at different time points.

In one embodiment, the method for detecting a target substance in tears comprises the following steps:

a. contacting a first absorbent sheet with the ocular surfaces of at least two different subjects to collect tears, so as to give at least two first absorbent sheets at least partially saturated with tears, wherein the first absorbent sheet at least partially saturated with tears has a fully saturated portion of a predetermined length or is fully saturated with tears;

b. contacting the fully saturated portion of the predetermined length of each of the first absorbent sheets with a sample elution solution of a predetermined volume for a predetermined time to give a tear dilution solution, or immersing the first absorbent sheet fully saturated with tears in the sample elution solution of the predetermined volume to give a tear dilution solution; and

c. using the obtained tear dilution solution separately for immunological detection to analyze the target substance in the tears collected from the different subjects.

In one embodiment, the method for detecting a target substance in tears comprises the following steps:

a'. contacting at least two first absorbent sheets separately with the ocular surfaces of different subjects at different time points to collect tears, so as to give at least two first absorbent sheets at least partially saturated with tears, wherein the first absorbent sheet at least partially saturated with tears has a fully saturated portion; and

b'. contacting the at least two first absorbent sheets separately with the immunological detection card according to the third aspect of the present disclosure to analyze the target substance in the tears.

In one embodiment, the different subjects are in the same or similar state, e.g., in a healthy state or suffering from the same or similar diseases. In one embodiment, the different subjects are in different states, e.g., in a healthy state or suffering from the same or similar diseases. In one embodiment, provided herein is a qualitative, semi-quantitative, or quantitative detection of a target substance in one or more tears from different subjects. In one embodiment, provided herein is a quantitative detection of a target substance in a plurality of tears from different subjects.

In one embodiment, the method for detecting a target substance in tears comprises the following steps:

a. contacting a first absorbent sheet with the ocular surface to collect tears, so as to give a first absorbent sheet at least partially saturated with tears;

b. cutting a fully saturated portion of a predetermined length from the first absorbent sheet at least partially saturated with tears;

c. contacting the fully saturated portion of the predetermined length with a sample elution solution of a predetermined volume for a predetermined time;

d. collecting the sample elution solution after the contact with the first absorbent sheet to give a tear dilution solution; and

e. using the tear dilution solution collected in step d for immunological detection to analyze the target substances in the tears.

In one embodiment, the method for detecting a target substance in tears comprises the following steps:

a. contacting a first absorbent sheet with the ocular surface to collect tears, so as to give a first absorbent sheet at least partially saturated with tears, wherein the first absorbent sheet at least partially saturated with tears has a fully saturated portion of a predetermined length;

b. contacting the fully saturated portion of the predetermined length with a sample elution solution of a predetermined volume for a predetermined time;

c. collecting the sample elution solution after the contact with the first absorbent sheet to give a tear dilution solution;

and

d. using the tear dilution solution collected in step c for immunological detection to analyze the target substances in the tears.

In one embodiment, provided herein is a qualitative, semi-quantitative, or quantitative detection of different target substances. In one embodiment, provided herein is a qualitative, semi-quantitative, or quantitative detection of at least about 2, 5, 10, 20, 50, 100, 200, or more different target substances in the same tear sample. In one embodiment, the same tear sample is a small-volume sample. For example, the amount of tear samples is less than about 0.0050 mL, preferably less than about 0.0040 mL, more preferably less than 0.0030 mL, and most preferably less than 0.0025 mL. In one embodiment, provided herein is a quantitative and comparative detection of at least about 2, 5, 10, 20, 50, 100, 200, or more different target substances in the same tear sample. In one embodiment, provided herein is a detection method for comparing the relative amounts of at least about 2, 5, 10, 20, 50, 100, 200, or more different target substances in the same tear sample.

In a sixth aspect, provided herein is a tear sample collection and treating device, comprising:

a first package comprising a first absorbent sheet, and
a second package comprising a sample elution solution,
wherein the sample elution solution comprises a buffering agent, a surfactant, and a stabilizer, and has a pH value of 7.0 to 9.0.

In one embodiment, the sample elution solution comprises water as a solvent; the sample elution solution further comprises a preservative and/or a tonicity adjusting agent.

In one embodiment, the sample elution solution comprises water as a solvent, a buffering agent, a surfactant, a stabilizer, a preservative, and a tonicity adjusting agent, and has a pH value of 7.0 to 9.0. In one embodiment, the first absorbent sheet, the sample elution solution, the buffering agent, the surfactant, the stabilizer, the preservative, and the tonicity adjusting agent are as defined in the first aspect.

In a seventh aspect, further provided herein is use of the tear sample collection and treating device according to the sixth aspect in collecting and treating tears.

In an eighth aspect, further provided herein is an immunological sampling and detection kit, comprising the first package and the second package according to the sixth aspect and an immunological detection card; preferably, the immunological detection card is the immunological detection card according to the third aspect described above.

Those skilled in the art will appreciate that the features of any aspect, embodiment, or example of the present disclosure may be combined, provided that there is no conflict or incompatibility between them.

Beneficial Effects of Present Disclosure

The method provided by the present disclosure enables the use of a fixed length or full length of an absorbent sheet, particularly a typical Schirmer tear detection filter paper strip, as a device for collecting tear samples, allowing for quantitative collection. The tear samples can be reproducibly and stably released in a quantitative manner after elution with the sample elution solution provided by the present disclosure. In addition, the tear sample collection and treating device of the present disclosure significantly improves the convenience, accuracy, and stability of subsequent detection compared to existing tear collection and treating methods. Furthermore, the obtained tear dilution solution can be directly used for detecting target substances in tears on platforms such as fluorescent immunochromatography, colloidal gold immunochromatography, latex immunochromatography, making the sampling and detection process more convenient and reducing operational difficulty and cost.

BRIEF DESCRIPTION OF THE DRAWING

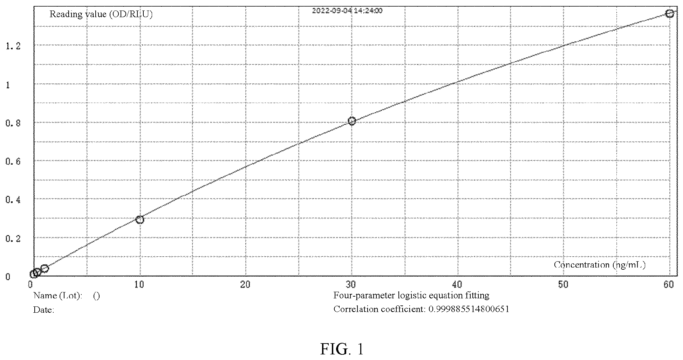

FIG. 1 shows the calibration curve from Example 5 of the present disclosure.

Examples

The preparation method of the present disclosure will be further illustrated in detail with reference to the following specific examples. It will be appreciated that the following examples are merely exemplary illustrations and explanations of the present disclosure and should not be construed as limiting the claimed scope of the present disclosure. All technical solutions implemented on the basis of the content of the present disclosure are encompassed within the claimed scope of the present disclosure.

Unless otherwise indicated, the experimental methods used in the following examples are all conventional methods in the art; the reagents, starting materials, instruments, devices used in the following examples are commercially available.

Example 1 Consistency test of absorbent sheets

20 commercially available detection filter paper strips for the Schirmer tear secretion test were provided, and a portion of the same length was cut from each strip. The weight of each cut portion was measured using a precise analytical balance, and the data were recorded as the dry weight. After weighing, each cut portion was then fully soaked with tears and immediately weighed again using the precise analytical balance, with the data recorded as the wet weight. The mass of the absorbed tears was obtained by subtracting the dry weight from the wet weight of each cut portion. Assuming a tear specific gravity of 1 (i.e., a density of 1 g/mL), the volume of the absorbed tears was calculated. The results are shown in Table 1 below.

**Table 1**

| Serial number | 1 | 2 | 3 | 4 | 5 | 6 | 7 | 8 | 9 | 10 |
|---|---|---|---|---|---|---|---|---|---|---|
| Dry weight (g) | 0.0016 | 0.0018 | 0.002 | 0.0017 | 0.0018 | 0.002 | 0.0017 | 0.0019 | 0.0017 | 0.0018 |
| Wet weight (g) | 0.0035 | 0.0038 | 0.004 | 0.00365 | 0.00369 | 0.00399 | 0.00368 | 0.0039 | 0.0037 | 0.00377 |
| Wet weight - dry weight (g) | 0.0019 | 0.002 | 0.002 | 0.00195 | 0.00189 | 0.00199 | 0.00198 | 0.002 | 0.002 | 0.00197 |
| Mass of absorbed liquid (g) = volume (mL) | 0.0019 | 0.002 | 0.002 | 0.00195 | 0.00189 | 0.00199 | 0.00198 | 0.002 | 0.002 | 0.00197 |
| Serial number | 11 | 12 | 13 | 14 | 15 | 16 | 17 | 18 | 19 | 20 |
| Dry weight (g) | 0.0019 | 0.0017 | 0.0019 | 0.0018 | 0.0019 | 0.0018 | 0.0018 | 0.002 | 0.0017 | 0.0017 |
| Wet weight (g) | 0.00386 | 0.00368 | 0.00389 | 0.0038 | 0.004 | 0.004 | 0.0041 | 0.0041 | 0.00363 | 0.00366 |
| Wet weight - dry weight (g) | 0.00196 | 0.00198 | 0.00199 | 0.002 | 0.0021 | 0.0022 | 0.0023 | 0.0021 | 0.00193 | 0.00196 |
| Mass of absorbed liquid (g) = volume (mL) | 0.00196 | 0.00198 | 0.00199 | 0.002 | 0.0021 | 0.0022 | 0.0023 | 0.0021 | 0.00193 | 0.00196 |
| Mean volume of absorbed liquid | 0.00201 | | | | | | | | | |
| Volume deviation of absorbed liquid | 9.80333E-05 | | | | | | | | | |
| Volume CV of absorbed liquid | 5% | | | | | | | | | |

**As can be seen from Table 1 above, the use of commercially available detection filter paper strips for the Schirmer tear secretion test with a predetermined length gave a deviation of $9.8 \times 10^{-5}$ and a coefficient of variation of 5%. Therefore, the volume stability of the obtained tear samples was validated to lay a foundation for the detection of samples by quantitative detection kits and for the quantitative detection of target substance concentrations in tears through standard curve regression.**

Example 2 Preparation of fluorescent immunochromatographic detection card

1. Preparation of blotting membrane: A blank blotting membrane with a control line and a detection line was dried at 37 °C for 8-20 h.

2. Preparation of sample receiving pad: 0.001 g of fluorescent latex microspheres were added to 0.9 mL of a first washing solution containing 10.66 g/L of morpholinoethanesulfonic acid having a pH of about 6.1. The mixture was well mixed by ultrasonication and then centrifuged at 10,000 rpm to 15,000 rpm for 30 min. The supernatant was discarded. 0.12 mL of a 20 mg/mL solution of sulfo-NHS in the first washing solution and 0.06 mL of 20 mg/mL 1-ethyl-(3-dimethylaminopropyl)carbodiimide hydrochloride were added sequentially to the residue. The mixture was activated for 30 min at 35-40 °C by stirring at 100-150 rpm, followed by a centrifugation for 20 min with the rotation speed set at 10,000-15,000 rpm. The supernatant was discarded. 1 mL of a 5.96 g/L N-(2-hydroxyethyl)piperazine-N-2-ethanesulfonic acid solution at pH 8.0 was added to the residue. The mixture was ultrasonicated until the solution became clear, and then centrifuged for 20 min. The supernatant was discarded. 0.0001 g of a murine anti-human matrix metalloproteinase-9 IgG was added to the residue, and the coupling reaction was continued after the mixture was well mixed. The obtained reaction mixture was centrifuged for 30 min, and the supernatant was discarded. 1 mL of a blocking solution containing 30 g/L of casein, 10 g/L of bovine serum albumin, and 0.05 mol/L of Trisbase buffer was added to the residue after the coupling reaction for blocking. The mixture was centrifuged for 20 min, and the supernatant was discarded. 1 mL of Trisbase buffer at pH 8.0 was added. The mixture was ultrasonicated until the solution became clear, and then centrifuged for 20 min. The supernatant was discarded. Then, 1 mL of a solution containing 30% of sucrose, 0.5% of casein, 1% of glycine, and 50 mM of a Trisbase at pH 8.0 was added to the residue for final volume adjustment, so as to give a mixture. The mixture was sprayed onto a glass fiber membrane at an amount of 5-10 μL/cm to give a sample receiving pad.

3. The sample receiving pad prepared in the previous step was placed in an overlapping manner at the second end of the blotting membrane proximal to the detection line;

4. An absorbent pad was placed in an overlapping manner at the first end of the blotting membrane proximal to the control line.

Example 3 Preparation of colloidal gold detection card

1. Preparation of blotting membrane: A blank blotting membrane with a control line and a detection line was dried at 37 °C for 8-20 h.

2. Preparation of sample receiving pad: 1 mL of 40 nm colloidal gold solution was taken, and 10 μL of 50 mM potassium carbonate was added thereto. The mixture was well mixed by vortex and left to stand for 5 min. 10 μg of a murine anti-human matrix metalloproteinase-9 IgG was taken and directly added to the colloidal gold solution, and the mixture was well mixed by vortex and left to stand for reaction at room temperature for 30 min. After the reaction was completed, 100 μL of 10% BSA (bovine serum albumin) was added, and the mixture was well mixed by vortex and left to stand for reaction at room temperature for 30 min. After the reaction was completed, a centrifugation was performed at 10,000 rpm for 20 min, and the supernatant was discarded. Then, 0.1 mL of a solution containing 30% of sucrose, 0.5% of casein, 1% of glycine, and 50 mM of a Trisbase at pH 8.0 was added to the residue for final volume adjustment, so as to give a mixture. The mixture was sprayed onto a glass fiber membrane at an amount of 5-10 μL/cm to give a sample receiving pad.

3. The sample receiving pad prepared in the previous step was placed in an overlapping manner at the second end of the blotting membrane proximal to the detection line;

4. An absorbent pad was placed in an overlapping manner at the first end of the blotting membrane proximal to the control line.

Example 4 Test of sample elution rate

100 μL of a calibrator was pipetted and added to the sample loading well of the detection card. The card was placed horizontally and allowed to react in the dark for 15 min before reading the results. The calibrators provided were solutions of MMP-9 (matrix metalloproteinase-9) ranging from S0 to S5, with concentrations sequentially set at 0 ng/mL, 0.3 ng/mL, 1 ng/mL, 10 ng/mL, 30 ng/mL, and 60 ng/mL.

The calibrators with different concentrations described above were separately loaded onto the detection cards prepared in Examples 2 and 3 to verify their accuracy. The results are shown in Tables 2 and 3 below. The C value is the signal value of the control line, the T value is the signal value of the detection line, and T/C is the ratio of the detection line to the control line. CV is the detection results of the coefficient of variation difference, and the coefficient of variation difference was within 10%, indicating stable tear releases.

Table 2

| Sample | T/C | T | C | CV % | Sample | T/C | T | C | CV % |
|---|---|---|---|---|---|---|---|---|---|
| S0 | 0.00882 | 580 | 65293 | 27 | S3 | 0.281405 | 19909 | 70749 | 3 |
| | 0.013085 | 835 | 63782 | | | 0.294866 | 18964 | 64315 | |
| | 0.008417 | 630 | 74816 | | | 0.304813 | 21095 | 69210 | |
| | 0.007161 | 560 | 78147 | | | 0.291391 | 18088 | 62073 | |
| S1 | 0.020852 | 1618 | 65096 | 4 | S4 | 0.738208 | 53496 | 72468 | 6 |
| | 0.019055 | 1152 | 71732 | | | 0.823008 | 68531 | 83269 | |
| | 0.019435 | 1300 | 66915 | | | 0.812105 | 60367 | 74334 | |
| | 0.019362 | 1350 | 6800 | | | 0.851616 | 72206 | 84787 | |
| S2 | 0.039459 | 3079 | 78039 | 5 | S5 | 1.364312 | 78523 | 57555 | 2 |
| | 0.040404 | 2534 | 62718 | | | 1.383192 | 89675 | 64832 | |
| | 0.03613 | 2209 | 61149 | | | 1.327937 | 114823 | 86467 | |
| | 0.039423 | 2760 | 70008 | | | 1.38649 | 111027 | 80077 | |

Table 3

| Sample | T | C | Sample | T | C |
|---|---|---|---|---|---|
| S0 | - | ++++ | S3 | ++ | ++++ |
| | - | ++++ | | ++ | ++++ |
| | - | ++++ | | ++ | ++++ |
| | - | ++++ | | ++ | ++++ |
| S1 | + | ++++ | S4 | +++ | ++++ |
| | + | ++++ | | +++ | ++++ |
| | + | ++++ | | +++ | ++++ |
| | + | ++++ | | +++ | ++++ |
| S2 | + | ++++ | S5 | +++ | ++++ |
| | + | ++++ | | +++ | ++++ |
| | + | ++++ | | +++ | ++++ |
| | + | ++++ | | +++ | ++++ |

Example 5 Tear detection test (dilution method)

A commercially available detection filter paper strip for the Schirmer tear secretion test was brought into contact with the ocular surface of a subject to collect tears, allowing the filter paper strip to be saturated with tears. According to the procedures in Example 1, a portion of the detection filter paper strip of the same length fully saturated with tears was cut and immersed in 200 μL of sample elution solution for 3 s of elution. Then, the eluted liquid was collected to give a tear dilution solution, which was loaded onto the sample receiving pad of the detection card prepared in Example 2 for analysis. The C value is the signal value of the control line, the T value is the signal value of the detection line, and T/C is the ratio of the detection line to the control line. The concentration was calculated according to the calibration curve. Five tests were performed for each of two different subjects. The results are shown in Tables 4 and 5 below.

The calibration curve was fitted using a four-parameter logistic equation, defined as follows:

$$Y = (A - D) / [1 + (X/C)^\wedge B] + D$$

A = 4.63846376754392
B = -1.00725333313379
C = 143.60632119348
D = 0.00936819607330606

Correlation coefficient R2: 0.999885514800651

Table 4. Detection and calculation data for subject 1

| | Number of detections | Result | | | |
|---|---|---|---|---|---|
| | | T | C | T/C | MMP-9 concentration [ng/mL] |
| Subject 1 | 1 | 19800 | 66915 | 0.295897781 | 9.66269 |
| | 2 | 20100 | 68000 | 0.295588235 | 9.65164 |
| | 3 | 19990 | 78039 | 0.256153974 | 8.25634 |
| | 4 | 18970 | 62718 | 0.302465002 | 9.89741 |
| | 5 | 19007 | 65096 | 0.291984146 | 9.52313 |

Table 5. Detection and calculation data for subject 2

| | Number of detections | Result | | | |
|---|---|---|---|---|---|
| | | T | C | T/C | MMP-9 concentration [ng/mL] |
| Subject 2 | 1 | 15872 | 74816 | 0.212147134 | 6.72661 |
| | 2 | 16500 | 78147 | 0.211140543 | 6.69195 |
| | 3 | 15600 | 65096 | 0.239646061 | 7.67918 |
| | 4 | 15980 | 71732 | 0.222773658 | 7.09342 |
| | 5 | 16002 | 66915 | 0.239139206 | 7.66153 |

Example 6 Tear detection test (sandwich method)

A commercially available detection filter paper strip for the Schirmer tear secretion test was brought into contact with the ocular surface to collect tears, allowing the filter paper strip to be saturated with tears. The filter paper strip was placed on the sample receiving pad of the kit prepared in Example 3 for analysis. The results are shown in Tables 6 and 7 below.

Table 6

| | Number of detections | Result | | |
|---|---|---|---|---|
| | | T | C | MMP-9 |
| Subject detection | 1 | '++ | '++++ | Positive |
| | 2 | '++ | '++++ | Positive |
| | 3 | '++ | '++++ | Positive |
| | 4 | '++ | '++++ | Positive |
| | 5 | '++ | '++++ | Positive |

Table 7

| | Number of detections | Result | | |
|---|---|---|---|---|
| | | T | C | MMP-9 |
| Subject 2 detection | 1 | '+++ | '++++ | Positive |
| | 2 | '+++ | '++++ | Positive |
| | 3 | '+++ | '++++ | Positive |
| | 4 | '+++ | '++++ | Positive |
| | 5 | '+++ | '++++ | Positive |

The embodiments of the present disclosure have been described above, which, however, are not intended to limit the present disclosure. Any modification, equivalent replacement, improvement made without departing from the spirit and principle of the present disclosure shall fall within the protection scope of the present disclosure.

**Claims**

1. A tear sample elution solution, comprising: water as a solvent, a buffering agent, a surfactant, a stabilizer, a preservative, and a tonicity adjusting agent, wherein the tear sample elution solution has a pH value of 7.0 to 9.0.

2. The tear sample elution solution according to claim 1, wherein the buffering agent is selected from an organic amine buffering agent, preferably an organic amine buffering agent with a hydroxyl group, and more preferably tris(hydroxymethyl)aminomethane.

3. The tear sample elution solution according to claim 1 or 2, wherein the surfactant comprises a first surfactant and a second surfactant different from the first surfactant; preferably, the surfactant is selected from a nonionic surfactant.

4. The tear sample elution solution according to claim 3, wherein the first surfactant is selected from a polysorbate surfactant; the second surfactant is selected from a block polyether surfactant, preferably a polyethylene glycol surfactant with a terminal hydroxyl group, or a polymer surfactant composed of methyl oxirane, 1,2-ethylenediamine, and oxirane.

5. The tear sample elution solution according to any one of claims 1 to 4, wherein the stabilizer is selected from albumin, casein, or gelatin.

6. The tear sample elution solution according to any one of claims 1 to 5, wherein the preservative is selected from Proclin-300, sodium azide, or thiomersal.

7. The tear sample elution solution according to any one of claims 1 to 6, wherein the sample elution solution may comprise about 10 mM to 100 mM of a buffering agent, about 0.1 wt% to 5 wt% of a surfactant, about 0.1 wt% to 5 wt% of a stabilizer, and optionally about 0.01 wt% to 0.5 wt% of a preservative and about 0.1 wt% to 5 wt% of a tonicity adjusting agent; preferably, the elution solution has a pH value of about 7.0 to 9.0;
preferably, the sample elution solution comprises about 50 mM of a Tris buffering agent, about 0.5% of TWEEN 20, about 2.5 wt% of Triton X-100, about 1 wt% of casein, and about 0.2 wt% of Proclin-300 and about 0.9 wt% of NaCl, and has a pH value of about 7.4.

8. A method for preparing a tear dilution solution, comprising the following steps:

a. contacting a first absorbent sheet with the ocular surface to collect tears, so as to give a first absorbent sheet at least partially saturated with tears, wherein the first absorbent sheet at least partially saturated with tears has a fully saturated portion of a predetermined length or is fully saturated with tears; and
b. contacting the fully saturated portion of the predetermined length of the first absorbent sheet with the sample elution solution according to any one of claims 1 to 7 of a predetermined volume for a predetermined time to give a tear dilution solution, or immersing the first absorbent sheet fully saturated with tears in the sample elution solution according to any one of claims 1 to 7 of the predetermined volume to give a tear dilution solution;

wherein preferably, the predetermined time for contacting the first absorbent sheet with the sample elution solution is 1 s to 5 min, preferably 3 s to 3 min, and more preferably 5 s to 1 min.

9. A tear sample collection and treating device, comprising:

a first package comprising a first absorbent sheet, and
a second package comprising a sample elution solution,
wherein the sample elution solution comprises a buffering agent, a surfactant, and a stabilizer, and has a pH value of 7.0 to 9.0, wherein the buffering agent, the surfactant, and the stabilizer are as defined in any one of claims 1 to 7;
preferably, the sample elution solution is the sample elution solution according to any one of claims 1 to 7.

10. Use of the tear sample collection and treating device according to claim 9 in collecting and treating tears.

FIG. 1

## INTERNATIONAL SEARCH REPORT

| International application No. |
|---|
| **PCT/CN2023/122840** |

| A. | CLASSIFICATION OF SUBJECT MATTER |
|---|---|

G01N1/14(2006.01)i

According to International Patent Classification (IPC) or to both national classification and IPC

| B. | FIELDS SEARCHED |
|---|---|

Minimum documentation searched (classification system followed by classification symbols)

IPC: G01N

Documentation searched other than minimum documentation to the extent that such documents are included in the fields searched

Electronic data base consulted during the international search (name of data base and, where practicable, search terms used)

CNABS; CNTXT; VEN; JPABS; USTXT; EPTXT; WOTXT; CNKI; Web of Science; IEEE: 北京华视诺维医疗科技有限公司, 夏超然, 仲惟星, 张淼, 泪液, 洗脱液, 缓冲剂, 表面活性剂, 稳定剂, 防腐剂, 渗透压, 胺, 羟基, 叠氮化钠, 硫柳汞, tear, eluent, elutriant, buffer, buffer substance, buffering agent, surface active agent, surfactant, stabilizer, antiseptic, aseptic, preservative, osmotic pressure, amic, amine, hydroxy, hydroxyl, Proclin-300, sodium azide, merthiolate, thimerosal, thimersalate

| C. | DOCUMENTS CONSIDERED TO BE RELEVANT |
|---|---|

| Category* | Citation of document, with indication, where appropriate, of the relevant passages | Relevant to claim No. |
|---|---|---|
| X | CN 1767897 A (IQUUM INC.) 03 May 2006 (2006-05-03) claim 67, and description, page 1, last paragraph, page 2, paragraph 1, page 3, last paragraph, page 7, paragraph 2, page 8, last paragraph, page 9, paragraph 1, page 20, paragraph 3, and page 39, paragraph 2 | 1-10 |
| X | US 2010285596 A1 (WATERS TECHNOLOGIES CORP.) 11 November 2010 (2010-11-11) description, paragraphs [0050], [0069], [0150], and [0151] | 1-7 |
| X | CN 112739337 A (JOHNSON & JOHNSON SURGICAL VISION, INC.) 30 April 2021 (2021-04-30) description, paragraphs [0068]-[0076] | 1-7 |
| A | CN 113088411 A (SHANXI LIPUDA PHARMACEUTICAL TECHNOLOGY CO., LTD.) 09 July 2021 (2021-07-09) entire document | 1-10 |

☐ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **04 December 2023** | **11 December 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2023/122840**

| Patent document cited in search report | | | Publication date (day/month/year) | Patent family member(s) | | | Publication date (day/month/year) |
|---|---|---|---|---|---|---|---|
| CN | 1767897 | A | 03 May 2006 | CN | 1767897 | B | 02 March 2011 |
| US | 2010285596 | A1 | 11 November 2010 | None | | | |
| CN | 112739337 | A | 30 April 2021 | None | | | |
| CN | 113088411 | A | 09 July 2021 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2022112199260
- CN 2022112198906
- CN 2022112198893